# EUROPEAN PATENT APPLICATION

(11) **EP 0 566 198 A2**
(43) Date of publication of application: **20.10.1993**
(21) Application number: 93201054.9
(22) Date of filing: 13.04.1993
(51) Int. Cl.: C12Q 1/68, C12N 15/10, C12N 15/11, C12N 15/12

(54) **In vitro gene selection by means of RNA carriers**

(30) Priority: 13.04.1992 IT MI920892
(71) Applicant: ENICHEM S.p.A., I-20124 Milano (IT)
(72) Inventor: Rossi, Nicoletta, I-00100 Rome (IT)
(74) Representative: Fusina, Gerolamo

(57) **Abstract**

A method is disclosed for identifying and/or isolating unusual genic transcripts, based on the use of a special carrier in order to insert genic sequences, constituted by the product of transcription of yeast tRNA^{Leu}³ gene under the control by T7 phage RNA polymerase promoter.

## Description

The present invention relates to a method for identifying and/or isolating unusual genic transcripts, which methods is based on the use of a carrier obtained by processing a new ribonucleic acid (RNA) by means of genetic engineering procedures.

The method can be applied in particular to the identification and isolation of genic sequences transcribed with an extremely low frequency (<10⁻⁵ relatively to the total of transcribed messenger RNA) inside human cells, tissues and organs.

At present several techniques are known in order to isolate human genes by means of the production and selection of collections of recombinant genetic clones in microorganisms (Sambrook, Fritsch and Maniatis, Molecular Cloning, New York, 1989). Such methods require that the recombinant clones constituting a bank gene are replicated in vivo by growing individual bacterial or yeast colonies in a liquid medium or on culture slabs, the separation of the individual gene fragments and their selection by interaction with nucleotidic or proteinic probes chemically modified with radioactive, fluorescent or chemoluminescent derivatives.

However, in the case of not very abundant genic portions, the low efficiency of the above said procedures (and in particular, of the in vivo amplification) makes it necessary a large number of recombinant clones (>10⁸-10⁹) to be analysed, with high analytical costs and long analysis times. Only seldom the cloning of genes, the transcripts of which are poorely represented in cDNA collections, was obtained, as it may happen for some functions present in complex organs, such as, e.g., brain (Sutcliffe, Ann. Rev. Neurosci; 2: 157, 1988).

On the other hand, isolating and identifying the nucleotidic sequence of such genes is of basic importance in order to characterize the proteins such genes encode for. These may perform, in the most different tissues, structural, regulatory and enzymatic functions which are of basic importance for cellular physiology and usually are present at very low concentrations (Sutcliffe, Ann. Rev. Neurosci; 2: 157, 1988).

Knowing the structural and functional properties of these proteins is essential, because their alteration caused by factors of genetic or environmental character is the base of a large number of diseases for which neither timely diagnostic methodologies nor specific drugs or effective therapeutical applications are available at present.

The present Applicant succeded now in developing a novel method for in vitro identifying and/or isolating genes which are transcribed with low frequency and encode for unusual proteins, which method gave heretofore unhoped-for results.

In its widest aspect, the present invention relates to a method for genic sequences selection, which comprises:
-- the insertion of genic segments composing the bank genes, into a modifying gene encoding for a novel carrier RNA
-- the in vitro transcription of the constructed gene;
-- the in vitro selection of the molecules of carrier RNA containing specific genic inserts by means of oligodeoxynucleotidic probes;
-- the affinity purification of the selected carrier RNA molecules;
-- the enzymatic synthesis of DNA corresponding to selected molecules;
-- the amplification and sequentiation of such selected molecules;

with said method being characterized in that as the carrier a ribonucleic acid (RNA) is used which is constituted by the product of transcription of yeast tRNA^{Leu3} gene placed under the control by the promoter of RNA polymerase of T7 phage (Otsuka et al., Mol. Cell. Biol. 1: 269, 1981; Baldi et al., Science 255: 1404, 1992).

The method according to the present invention results to be much simpler, quicker and more effective than the methods used to date, based on construction and selection of recombinant bank genes in microorganisms and makes it possible the technological limits of these methods to be overcome.

The invention proved to be very effective for the purposes of locating and isolating genic sequences transcribed with an extremely low frequency in human cells, tissues and organs.

In particular, the method proposed by the present invention comprises the following steps:
-- construction of a gene encoding for a novel carrier RNA obtained by modifying yeast tRNA^{Leu3} gene and insertion of the genic segments composing bank genes from human organs and tissues;
-- in vitro synthesis of carrier RNA by means of the transcription of the constructed gene by specific RNA polymerase and enzymatic circularization of carrier RNA;
-- in vitro selection of molecules of carrier RNA containing specific genic inserts, by interaction with oligodeoxynucleotidic probes and enzymatic linearisation of the resulting RNA-DNA heterocomplexes by means of RNase H;
-- affinity purification of selected carrier RNA molecules, enzymatic synthesis of corresponding DNA and amplification thereof by cyclic synthesis by means of heat-stable polymerases;
-- sequentiation and analysis of amplified molecules.

As previously said, the present method implies inserting genic segments derived from bank genes of human organs and tissues into a gene encoding for a novel carrier RNA. The size of the genic fragments is generally of from 500 to 1,500 bp.

The operations on DNA were carried out according to the usual techniques as adopted in customary practice (Sambrook, Fritsch and Maniatis, Molecular Cloning, New York, 1989).

The genic segments from human cDNA bank genes are inserted into the Hpa I site of the intron of yeast pre-tRNA^{Leu3} gene in plasmide pGEM-1, under the control by the promoter of T7 phage RNA polymerase (Otsuka et al., Mol. Cell. Biol. 1: 269, 1981; Baldi et al., Science 255: 1404, 1992).

The plasmid is then linearised downstream of 3' end of pre-tRNA^{Leu3} gene by restriction with Xho I endonuclease and is transcribed in vitro by T7 RNA polymerase, as described by Baldi and co-workers (Baldi et al., Science 255: 1404, 1992). In that way from 1 to 5 micrograms of transcribed linear RNA are obtained in vitro.

The obtained transcripts retain the structural properties of native pre-tRNA and in particular are correctly cleft by the Xenoeus splicing endonuclease (Otsuka et al., Mol. Cell. Biol. 1: 269, 1981: Mattoccia et al., Cell. 55: 731, 1988), an enzyme which specifically recognizes the structural characteristics of pre-tRNA.

Said pre-tRNA is hence useable as carrier RNA for inserting genic segments of size of from 500 to 1,500 bp, while simultaneously retaining a correct structural configuration, in particular as regards the formation of a correct complementary coupling in the acceptor region.

Such a coupling makes it possible a correct space proximity of 5' and 3' terminal ends of the molecules of transcribed RNA and their in vitro circularization by T4 phage RNA ligase to be obtained according to the procedures as reported by Uhlenbeck and co-workers (Pan et al., Science 254: 1361, 1991).

The efficiency of the circularization reaction is verified, after labeling the substrate by transcription in the presence of ³²P-UTP, by means of the specific digestion of circularized pre-tRNA with RNase P of Xenopus (Carrara et al., Cell. 58: 37, 1989) and is quantified, after removing the circularized molecules by polyacrylamide or agarose gel electrophoresis, with yields of the order of 75%.

The selection of circular molecules of carrier RNA containing individual genic inserts is carried out by hybridisation to specific synthetic oligodeoxynucleotides and subsequent site-specific linearization at the site of formation of heterocomplex RNA-DNA in the presence of RNase H (Grossman, Proc. Natl. Acad. Sci USA 70: 3339, 1973).

The reaction efficiency, calculated in the presence of an excess of non-specific transcribed RNA, results to be of the order of 10⁻⁴-10⁻⁵ and is hence compatible for the in vitro selection of unusual genic transcripts, the frequency of which in collections of cDNA from human organs is estimated to be of approximately 10⁻⁵ (Sutcliffe, Ann. Rev. Neurosci, 2: 157, 1988).

The selected molecules are modified by means of the enzymatic synthesis of a polyadenosine sequence at 3' terminal end of linear RNA molecules in the presence of polyA-polymerase (Gethin et al., Nature 287: 301, 1980).

The genic inserts in the selected carrier RNA molecules are amplified, after synthesis of single-stranded complementary DNA by reverse transcriptase (Okayama et al., Methods Enzymol. 154: 3, 1987) through PCR cyclic synthesis (Saiki et al., Science 239: 487, 1988), using, as specific primers, polythymidine and an oligonucleotide corresponding to intron sequences adjacent to the site of insertion of carrier pre-tRNA into the gene.

The nucleotidic sequence of the amplified genic segments is determined by automated sequentiation and the aminoacidic sequence of expressed proteins is deduced and analysed by means of specific computer-supported programmes (Bishop and Rawlings, Nucleic Acid and Protein Sequence Analysis, Oxford, 1987).

In order to demonstrate the development disclosed by the present invention the following example is reported. The following example is anyway supplied for merely illustrative purposes and in no way should be construed as being limitative of the purview of the present invention, as specified in the appended claims.

### Example 1

### Method for the selection of the genes which encode for human adrenergic receptors.

Nco I-Sma I and Pst I-Pst I fragments (of size of 1,062 and 948 bp, respectively) from cDNA of genes alpha2C4 and alpha2C10 modifying human alpha2-adrenergic receptors are ligated to the single Hpa I site of the intron of pre-tRNA^{Leu3} gene of yeast, inserted into plasmid pGEM-1, under the control of T7 phage RNA polymerase promoter.

Linear RNA (1-5 micrograms) is obtained in vitro after restriction of constructed gene by means of Xho I endonuclease and subsequent transcription by T7 RNA polymerase (120 U) for 90 minutes, 370C, in a reaction volume of 40 microlitres, in the presence of 2,5 mM GMP.

The obtained transcripts retain the structural properties of native pre-tRNA and in particular are correctly cleft the splicing endonuclease of Xenopus, an enzyme which specifially recognizes the structural characteristics of pre-tRNA.

The reaction of circularization of transcribed RNA is performed in the presence of T4 ligase (18 U) and substrate RNA (0.2-1 microgram), over 120 minutes, 370C, in a reaction volume of 10 microlitres.

The efficiency of the circularization reaction is verified, after labeling the substrate by transcription in the presence of alpha-³²P-UTP, by means of the specific digestion of circularized pre-tRNA with RNase P of Xenopus according to the procedures currently in use (Carrara et al., Cell. 58: 37, 1989).

The selection of circular molecules of carrier RNA containing individual genic inserts is carried out by hybridisation to specific synthetic oligodeoxynucleotides (2.5 pmol; length 24 nucleotides) over 3 minutes at 80°C and then over 10 minutes at 65°C, and subsequent site-specific linearisation at the site of hybridisation in the presence of RNase H (1U) over 60 minutes at 37°C, in a volume of 25 microlitres.

The selected molecules are modified by means of the enzymatic synthesis of a polyadenosine sequence at 3' terminal end of linear RNA molecules in the presence of polyA-polymerase according to described procedures (Cethin et al., Nature 287: 301, 1980).

The same methodology is used for the selection of cDNA collections from genic sequences transcribed in human brain and other human tissues.

## Claims

1. Method for genic sequences selection, which comprises:
-- insertion of genic segments composing the relevant bank genes, into a modifying gene encoding for a novel carrier RNA;
-- in vitro transcription of the constructed gene;
-- in vitro selection of molecules of carrier RNA by means of oligodeoxynucleotidic probes;
-- affinity purification of the selected carrier RNA molecules;
-- enzymatic synthesis of DNa corresponding to selected molecules;
-- amplification and sequentiation of selected molecules;
with said method being characterized in that as the carrier a ribonucleic acid (RNA) is used which is constituted by the transcription product of yeast tRNA^{Leu3} gene placed under the control by the promoter of RNA polymerase of T7 phage.
